(19) Europäisches Patentamt
European Patent Office
Office européen des brevets

(11) EP 4 659 857 A1

(12) EUROPEAN PATENT APPLICATION

(43) Date of publication:
10.12.2025 Bulletin 2025/50

(21) Application number: 24180709.8

(22) Date of filing: 07.06.2024

(51) International Patent Classification (IPC):
B01J 23/80 (2006.01)        B01J 21/18 (2006.01)
B01J 35/40 (2024.01)        B01J 37/02 (2006.01)
B01J 37/03 (2006.01)        B01J 37/18 (2006.01)
C07C 29/154 (2006.01)

(52) Cooperative Patent Classification (CPC):
(C-Sets available)
B01J 35/40; B01J 23/80; B01J 37/0201;
B01J 37/035; B01J 37/18; C07C 29/154;
B01J 21/18; B01J 2235/15; B01J 2235/30        (Cont.)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: Universitat Autónoma de Barcelona
08193 Bellaterra (Cerdanyola del Vallès)
Barcelona (ES)

(72) Inventors:
• Font Segura, Xavier
08193 Bellaterra (ES)
• Moral Vico, Antonio javier
08193 Bellaterra (ES)
• Ferrer Sanchez, Antonio
08193 Bellaterra (ES)
• Seyed Alireza, Vali
08193 Bellaterra (ES)

(54) **NOVEL CATALYST FOR METHANOL SYNTHESIS FROM CARBON DIOXIDE**

(57)    Catalyst containing ruthenium and zinc on a carbonaceous support for the synthesis of methanol from carbon dioxide. A process for preparing said catalyst involves the deposition-precipitation of dissolved metal salt precursors on biochar particles by adding a sodium carbonate precipitant solution. The solid catalyst precursor is separated and calcined and finally reduced in a hydrogen gas flow before the carbon dioxide hydrogenation.

Figure 2

(52) Cooperative Patent Classification (CPC): (Cont.)

C-Sets
**C07C 29/154, C07C 31/04**

## Description

FIELD OF THE INVENTION

The present invention relates to the field of catalysts used in the preparation of methanol by hydrogenation of carbon dioxide.

BACKGROUND ART

Increasing release of greenhouse gases such as carbon dioxide to the atmosphere as a consequence of the exploitation of fossil fuels is a major concern worldwide.

Hence, more research has been concentrated on carbon dioxide apprehension and transformation to value-added chemicals, as disclosed in Markewitz et al., Worldwide innovations in the development of carbon capture technologies and the utilization of CO2, Energy Environ. Sci., 2012, 5, 7281-7305.

Carbon dioxide capture and storage and chemical transformation have proved effectual techniques to cut down on carbon dioxide emissions and move towards renewable energy resources, as disclosed in Leung et al., An overview of current status of carbon dioxide capture and storage technologies, Renew. Sustain. Energy Rev., 2014, 39, 426-443.

In this regard, carbon dioxide hydrogenation to methanol is of great interest considering methanol as a potential fuel as well as a starting platform for the manufacture of numerous industrial chemicals, as disclosed in Jadhav et al., Catalytic carbon dioxide hydrogenation to methanol: A review of recent studies, Chem. Engin. Res. Design, 2014, 92, 2557-2567. However, such reaction typically takes place at extremely severe conditions in terms of temperature and pressure largely due to the carbon dioxide high thermodynamical stability and low reactivity, as disclosed in Raudaskoski et al., Catalytic activation of CO2: Use of secondary CO2 for the production of synthesis gas and for methanol synthesis over copper-based zirconia-containing catalysts, Catal. Today, 2009, 144, 318-323. Accordingly, researchers have been drawn to investigate potential solid catalysts which can catalyse the methanol synthesis reaction at milder conditions, as disclosed in Rohde et al., Membrane application in Fischer-Tropsch synthesis to enhance CO2 hydrogenation, Ind. Eng. Chem. Res., 2005, 44, 9653-9658.

For this purpose, many catalysts have been developed, among which Cu-based catalysts have been the most common ones.

In British patent application GB-A-1159035 it is disclosed the preparation of the catalyst $CuO/ZnO/Al_2O_3$ suitable for the synthesis of methanol from a mixture of hydrogen, carbon dioxide and carbon monoxide. Decades later, this catalyst is still used for the industrial synthesis of methanol, as disclosed in Zhang et al., Improved methanol synthesis performance of Cu/ZnO/Al2O3 catalyst by controlling its precursor structure, Green Energy Environ., 2022, 7, 722-781. It is further disclosed that the reaction conditions are at a temperature of 200-320 °C and at a pressure of 5-10 MPa.

In van den Berg et al., Structure sensitivity of Cu and CuZn catalysts relevant to industrial methanol synthesis, Nature Comm., 2016, 7, 1-7, it is disclosed the synthesis of several catalysts with copper particle sizes in the range from 2 to 15 nm on Zn-free supports (silica and carbon[high surface area graphite or carbon xerogel]), on supports containing zinc as Zn silicate or on supports incorporating Zn oxide. It also discloses the synthesis of $Cu/ZnO(Al_2O_3)$, and the use of the catalysts in the methanol synthesis reaction from synthesis gas, comprising (10% Ar, 7% $CO_2$, 23% CO and 60% $H_2$).

In the review article M. Behrens, Promoting the Synthesis of Methanol: Understanding the Requirements for an Industrial Catalyst for the Conversion of CO2, Angew. Chem. Int. Ed., 2016, 55, 14906-14908, it is disclosed that by using a combination of experimental, computational, and kinetic data, it was shown that the turnover frequency (TOF) for methanol critically depends on the coverage of the copper surface with metallic Zn atoms, wherein this surface alloy state is formed by a self-assembly process, through the migration of Zn species onto the copper surface.

Incorporation of additional metals to the catalyst has been proposed in the art.

In Toyir et al., Highly effective conversion of CO2 to methanol over supported and promoted copper-based catalysts: influence of support and promoter, Appl. Catal. B Environ., 2001, 29, 207-215, it is disclosed the preparation of gallium-promoted copper-based catalysts by impregnation methods on silica and ZnO supports, and their use for the hydrogenation of $CO_2$ to methanol. It is further disclosed that the use of hydrophobic silica enhanced the performance of the catalyst in terms of activity, selectivity, and stability.

In Chinese patent application CN-A-117085689, it is disclosed the preparation of a copper-based catalyst suitable for preparing methanol through hydrogenation of carbon dioxide, wherein the catalyst comprises the following components: Cu, ZnO, $ZrO_2$ or $Al_2O_3$ and C, and C is uniformly dispersed on the surface of the catalyst. The carbon source is selected from β-cyclodextrin, polyethylene glycol, ethylene glycol, glucose, or soluble starch, which is carbonized in the presence of the metallic components.

In Chinese patent application CN-A-116037132, it is disclosed the preparation of a catalyst for the synthesis of methanol through carbon dioxide hydrogenation, wherein the catalyst comprises Cu/Zn as active component, $Al_2O_3$ as carrier and $ZrO_2$ and/or CeOz as auxiliary agent.

In Chinese patent application CN-A-114920623, it is disclosed the preparation of a catalyst for the hydrogenation of carbon dioxide to obtain methanol, wherein the catalyst comprises $Cu/ZnO/Al_2O_3/Si$, which is obtained by silanization of the $Cu/ZnO/Al_2O_3$ precursor.

In Chinese patent application CN-A-114029063, it is disclosed the preparation of a catalyst for preparing methanol through hydrogenation of carbon dioxide, wherein the catalyst comprises metal copper, metal zinc, metal zirconium, a metal additive, selected from the group consisting of cerium, yttrium, aluminium, gallium, palladium, platinum, magnesium, manganese and chromium, and a carbon material, selected from the group consisting of silicon carbide, carbon nanofiber, activated carbon, carbon nanotube, graphite fibre, carbon nitride and carbon-based foam with a large specific surface area.

In Chinese patent application CN-A-113877573, it is disclosed the preparation of a catalyst for obtaining biofuel by hydrogenation of carbon dioxide, wherein the process comprises heating the biomass raw material to obtain a carbon material precursor; impregnating said carbon material precursor with a chemical activator, selected from sodium hydroxide, potassium hydroxide, phosphoric acid, and zinc chloride, and carbonizing it to obtain a mesoporous carbon carrier; loading said carrier with metal ions selected from copper, iron, cobalt, palladium, platinum, and silver; and calcining the loaded carbon carrier. According to the examples, it is disclosed that the methanol selectivity is in the range from 4% to 15%.

Thus, in spite of the different catalysts for preparing methanol by hydrogenation of carbon dioxide disclosed in the prior art, there is a need for further catalysts showing improved yield, higher selectivity and even higher stability.

SUMMARY OF INVENTION

An aspect of the invention is a process for preparing a catalyst.

Another aspect of the present invention relates to the catalyst obtainable according to that process.

Another aspect of the invention is a process for preparing methanol.

Another aspect of the invention is the use of said catalyst in the synthesis of methanol.

FIGURES

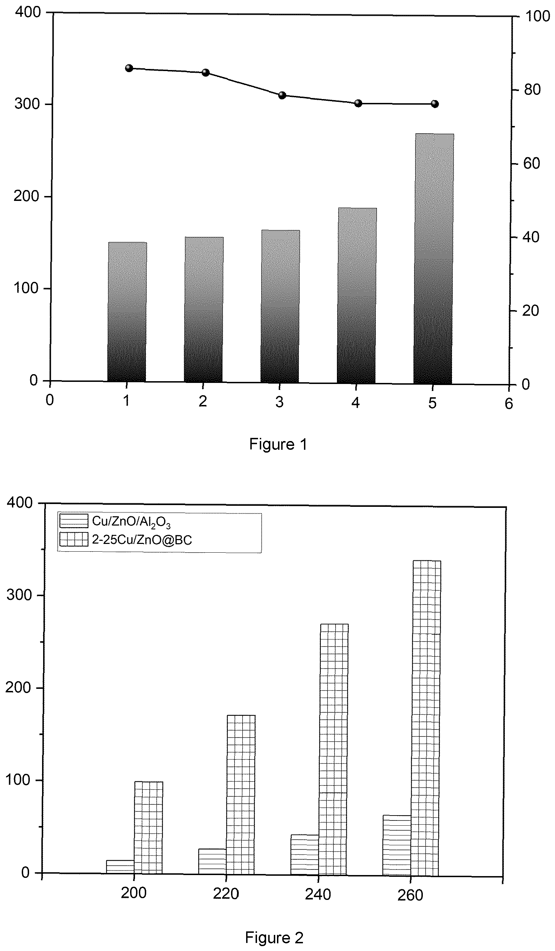

In Figure 1, in the left ordinate it is shown the methanol Space time yield (STY, expressed in $mg_{MeOH}g_{cat}^{-1}h^{-1}$), and in the right ordinate the Selectivity (expressed in %) represented for the catalyst 2-25%Cu/ZnO@BC at different Weight hourly space velocity (WHSV) (expressed in $mL\ g_{cat}^{-1}h^{-1}$) in the abscissa.

In Figure 2, in the ordinate it is shown the catalytic performance (STY, expressed in $mg_{MeOH}g_{cat}^{-1}h^{-1}$) of a catalyst of the invention (2-25%Cu/ZnO@BC) and the commercially available ternary catalyst $Cu/ZnO/Al_2O_3$, at different temperatures (200 °C, 220 °C, 240 °C, and 260 °C), 10 bar, and WHSV of 12000 mL $g_{cat}^{-1}h^{-1}$, shown in the abscissa. The catalyst of the invention proves to be significantly superior to the commercial one.

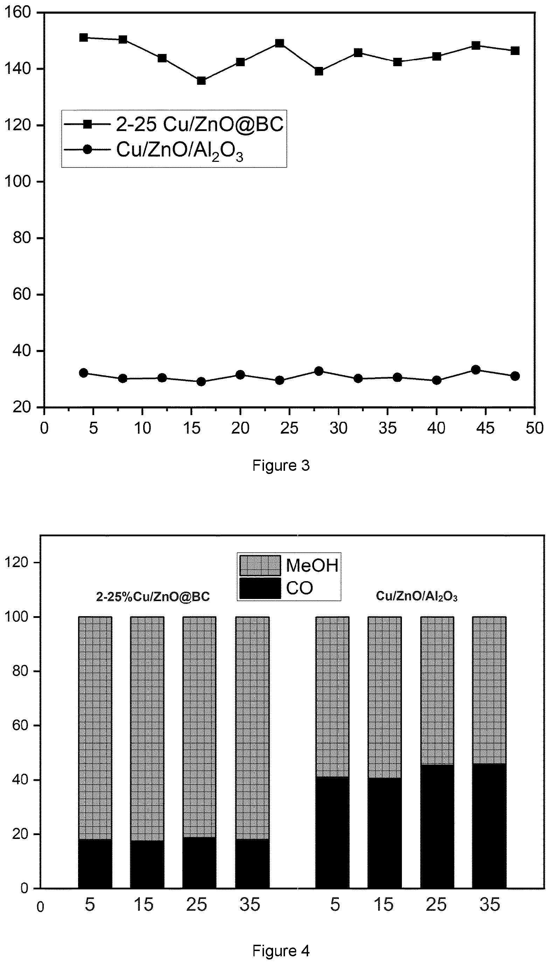

In Figure 3, in the ordinate it is shown the stability in the production of methanol (STY, expressed in $mg_{MeOH}g_{cat}^{-1}h^{-1}$) by a catalyst of the invention (2-25%Cu/ZnO@BC) and by the commercially available ternary catalyst $Cu/ZnO/Al_2O_3$ along the time, expressed in hours, which is represented in abscissa, up to 48 h. The catalyst of the invention maintains 97% of its catalytic activity after 44 h of catalytic reaction.

In Figure 4 it is shown the selectivity (ordinate, expressed in %) of a catalyst of the invention (2-25%Cu/ZnO@BC) and of the commercially available ternary catalyst $Cu/ZnO/Al_2O_3$ for producing methanol in comparison to the generation of carbon monoxide along the time on stream, expressed in hours, in the abscissa. The methanol selectivity of the catalyst of the invention is significantly higher than the selectivity exhibited by the commercially available catalyst, which decreases gradually after 25 h of reaction.

DESCRIPTION OF THE INVENTION

The present invention relates to a process for preparing a catalyst, which comprises:

1) dissolving a Cu (II) salt and a Zn (II) salt in water,
2) adding biochar (BC) particles to the solution obtained in step 1),
3) stirring the suspension obtained in step 2),
4) adding a precipitant solution to the stirred suspension obtained in step 3),
5) centrifuging and drying the solid obtained in step 4),
6) calcinating the solid obtained in step 5), and
7) reducing with hydrogen gas flow the calcinated solid particles obtained in step 6).

The inventors of the present invention have developed a process for preparing new catalysts, which surprisingly show higher yield and selectivity in the synthesis of methanol by hydrogenation of carbon dioxide than the commercially available ternary catalyst $Cu/ZnO/Al_2O_3$, which is used for the industrial production of methanol. Further, the catalyst of the invention exhibits a high stability because it maintains 97% of the catalytic activity after at least 44 h of catalytic reaction.

In the present description, as well as in the claims, the singular forms "a", "an" and "the" include the plural reference unless the context clearly indicates otherwise. The term "about" refers to a deviation of plus/minus 10%, preferably plus/minus 5%. The percentages are expressed in % by weight (wt%), unless stated the contrary. The ranges defined by the terms "between ... and ..." or by the terms "from ... to..." are meant to include also said stated endpoints thereof, and they also include any narrower sub-range.

In the present invention, the following definitions are used:

- NPs: catalyst in the form of Cu and ZnO nanoparticles
- STY: Space time yield, expressed in $mg_{MeOH}g_{cat}^{-1}h^{-1}$, is defined as quantity of product per unit volume and unit time, and calculated using the following equation:

$$CH_3OH\ STY\left(\frac{mg}{g_{cat}\times h}\right)=\left(\frac{Mass\ of\ methanol\ (mg)\ formed}{W_{Cu+Zn}(g)\times Hour}\right)$$

wherein $W_{Cu+Zn}$ is the weight of catalyst (g), including Cu, ZnO and BC.
- WHSV: Weight hourly space velocity, expressed in $mL.g_{cat}^{-1}.h^{-1}$, is defined as the quotient of the mass flow-rate of the gas divided by the mass of the catalyst in the reactor at the condition of standard temperature and pressure.
- Methanol selectivity: percentage of methanol formed over the reacted carbon dioxide, and calculated using the following equation

$$CH_3OH\ Selectivity\ (\%)=\left(\frac{moles\ of\ methanol\ formed}{n[CO_2]_{in}-n[CO_2]_{out}}\right)\times 100$$

wherein $n[CO_2]_{in}$ is the amount (in moles) of $CO_2$ at the inlet, $n[CO_2]_{out}$ stands for the amount of $CO_2$ at the outlet.
- n-m%Cu/ZnO@BC: catalyst comprising a molar ratio Cu:Zn of n:1, a m wt% of Cu +ZnO on the catalyst comprising biochar (BC)
- Carbon dioxide conversion ($X_{CO2}$) is calculated using the following equation:

$$X_{CO_2}(\%)=\left(\frac{n[CO_2]_{in}-n[CO_2]_{out}}{n[CO_2]_{in}}\right)\times 100$$

wherein $n[CO_2]_{in}$ is the amount (in moles) of $CO_2$ at the inlet, and $n[CO_2]_{out}$ stands for the amount of $CO_2$ at the outlet.

Process for preparing the catalyst

An aspect of the invention is a process for preparing a catalyst for the synthesis of methanol by hydrogenation of carbon dioxide.

The process for preparing the catalyst comprises:

1) dissolving a Cu(II) salt and a Zn salt in water,
2) adding biochar (BC) particles to the solution obtained in step 1),
3) stirring the suspension obtained in step 2),
4) adding a precipitant solution to the stirred suspension obtained in step 3),
5) centrifuging and drying the solid obtained in step 4),
6) calcinating the solid obtained in step 5), and
7) reducing with hydrogen gas flow the calcined solid obtained in step 6).

The process may include further steps related to a pre-treatment of the biochar, such as sieving and/or thermally treating.

In a preferred embodiment, the biochar is sieved to select particles ranging in size between 10 and 200 microns, preferably between 20 and 100 microns.

In a preferred embodiment, the particles of biochar are thermally treated, preferably at a temperature comprised between 400 °C and 800 °C, preferably between 500 and 700 °C, and more preferably about 600 ° C.

In a preferred embodiment, the biochar is sieved and thermally treated.

**Materials**

*Metal salts*

Cu(ll) salts suitable to be used in the process of the invention are Cu(ll) nitrate, and hydrates thereof. Preferably it is used Cu(ll) nitrate hydrate, and more preferably Cu(ll) nitrate 3 $H_2O$.

Zn salts suitable to be used in the process of the invention are Zn nitrate, and hydrates thereof. Preferably it is used Zn nitrate hydrate, and more preferably Zn nitrate 6 $H_2O$.

*Biochar*

Biochar (BC) is a porous, carbonaceous material that is produced by pyrolysis of biomass and is applied in such a way that the contained carbon remains stored as a long-term C sink or replaces fossil carbon in industrial manufacturing, as disclosed in EBC (2012-2023) 'European Biochar Certificate - Guidelines for a Sustainable Production of Biochar.' Carbon Standards International (CSI), Frick, Switzerland. (http://european-biochar.org). Version 10.3 from 5th Apr 2023.

Biochar is produced by biomass pyrolysis; a process whereby organic substances are broken down at temperatures ranging from 350 °C to 1000 °C in a low-oxygen process.

Biochar is commercially available from different suppliers, e.g., Carbo Culture (Finland), or Nova Carbo (Germany).

*Precipitant solution*

In an embodiment the precipitant solution is an aqueous solution of an alkali metal carbonate. In a preferred embodiment it is an aqueous solution of sodium carbonate.

The concentration of the precipitant is usually comprised between 0.01 M and 0.1 M, preferably between 0.02 M and 0.05 M, and more preferably it is 0.025 M.

**Steps**

*Dissolution of Cu(II) and Zn (II) salts*

In step 1) of the process of the invention, a Cu (II) salt and a Zn (II) salt are dissolved in water, preferably in ultrapure water.

Ultrapure water is water that has been purified to the highest levels of purity for all contaminant types, including: organic and inorganic compounds; dissolved and particulate matter; volatile and non-volatile; reactive, and inert; hydrophilic and hydrophobic; and dissolved gases. The most widely used requirements for ultrapure water quality are documented by ASTM D5127 "Standard Guide for Ultra-Pure Water Used in the Electronics and Semiconductor Industries" and SEMI F63 "Guide for ultrapure water used in semiconductor processing".

*Addition of biochar (BC) particles*

In step 2) of the process, biochar (BC) particles are added to the solution of the Cu (II) and Zn (II) salts.

*Stirring the suspension*

In step 3) of the process, the suspension of BC particles and Cu (II) and Zn (II) salts is stirred, preferably under vigorous stirring and, preferably, for a time usually about 2 h. In this step, without being bound by the theory, Cu (II) and Zn (II) species are generally adsorbed on the surface of BC particles.

*Addition of the precipitant solution*

In step 4) of the process, a precipitant solution, is added to the aqueous suspension formed by the dissolved metal salts and BC particles. In a preferred embodiment, the precipitant solution is added dropwise. In this step, the pH of the suspension is preferably adjusted to about 7.0. The addition of the precipitant solution triggers the formation of $Cu_xZn_y(OH)_m(CO_3)_n$ crystallites on the surface of BC particles, as disclosed in Zhang *et al., op. cit.*

In an embodiment, the suspension is allowed to age, between step 3) and step 4) in order to complete the formation of $Cu_xZn_y(OH)_m(CO_3)_n$ crystallites on the surface of BC particles.

In a preferred embodiment, the suspension is aged between step 3) and step 4). It is preferably maintained at a temperature comprised between 50 °C and 70 °C, preferably between 55 °C and 65 °C, and more preferably at 60 °C, and for a time comprised between 1 h and 5 h, preferably between 1.5 h and 4 h, and more preferably about 2 h.

*Centrifugation and drying of the solid*

In step 5) of the process, the suspension is centrifugated to isolate the solid.

In an embodiment, the suspension, prior to the centrifugation of step 5), is washed with water, preferably ultrapure water, several times, to remove the cations from the precipitant solution, preferably the sodium cation.

After the washing, the suspension is centrifugated to isolate the solid. The solid obtained in the centrifugation step is subsequently dried, preferably at a temperature comprised between 100 °C and 110 °C, and more preferably at 105 °C. Usually, the drying process is carried out overnight.

Particles comprising $Cu_xZn_y(OH)_m(CO_3)_n$@BC are obtained after the drying process.

*Calcination of the solid*

In step 6) of the process, particles comprising $Cu_xZn_y(OH)_m(CO_3)_n$@BC are calcinated.

The calcination takes place usually in a muffle furnace, typically at a temperature comprised between 300 °C and 500 °C, preferably between 350 °C and 450 °C, and more preferably at 400 °C, and for a period of time comprised usually between 1 h and 5 h, preferably between 2 h and 4 h, and more preferably 3 h.

*Reduction with hydrogen gas flow*

In step 7) of the process, the calcinated solid particles are reduced with hydrogen gas flow, typically using a pressure of 1 bar, and 20 sccm (standard cubic centimetres per minute). The reduction step takes place usually at a temperature comprised between 200 °C and 400 °C, preferably between 250 °C and 350 °C, and more preferably at 300 °C, and for a period of time comprised usually between 1 h and 4 h, preferably between 1.5 h and 3 h, and more preferably 2 h.

The samples of the catalyst, CuZnO@BC, are obtained and may be used for further characterization and for the methanol synthesis from carbon dioxide and hydrogen.

Catalyst

Another aspect of the present invention relates to a catalyst obtainable according to the process of the invention.

In the catalyst, Cu (II) is converted to Cu (0) by the reduction, and Zn is converted to ZnO.

The molar ratio Cu:Zn is comprised between 0.5:1 and 5:1, preferably between 1:1 and 4:1, more preferably between 1.5:1 and 3:1, and yet more preferably 2:1.

The content of Cu + ZnO in the catalyst is usually comprised between 5 wt% and 50 wt%, preferably between 10 wt% and 40 wt%, preferably between 15 wt% and 35 wt%, more preferably between 20 wt% and 30 wt%, more preferably between 22.5 wt% and 27.5 wt%, and yet more preferably the content is 25 wt%, over the total weight of the catalyst.

The content of BC in the catalyst is usually comprised between 50 wt% and 95 wt%, preferably between 60 wt% and 90 wt%, preferably between 65 wt% and 85 wt%, more preferably between 70 wt% and 80 wt%, more preferably between 72.5 wt% and 77.5 wt%, and yet more preferably the content is 75 wt%, over the total weight of the catalyst.

The catalyst of the invention may be characterized by scanning electron microscopy (SEM) equipped with an energy dispersive spectroscopy (EDS) detector.

This analytical tool may be used to determine the morphology, size distribution and elemental composition of the samples.

Nitrogen adsorption/desorption analyses may be carried out at -196 °C using an ASAP 2020 Micrometrics Inc (BET analyser). All samples were typically degassed at 80 °C for 20 h before analysis.

Crystalline structure of the catalyst samples may be investigated using Powder X-ray diffraction (XRD). The XRD patterns may be recorded in a diffractometer using Cu-K$\alpha$ radiation ($\lambda$=1.5418 Å). The measurements are typically made at room temperature at a range of 0°- 80° on 2$\theta$ with a step size of 0.02°. All XRD results may be analysed with the simulation of the nanoparticles obtained using the HighScore Plus software.

Scanning electron microscopy (SEM) images obtained for the calcined catalyst show that Cu/ZnO nanoparticles are well dispersed on the surface of the BC.

In addition, EDX elemental mapping images of the catalyst sample confirms the proper dispersion of Cu/ZnO as well as the excellent mixing of Cu and ZnO nanoparticles, which is critical for a synergistically active catalytic system as far as methanol synthesis from carbon dioxide hydrogenation is concerned.

Process for preparing methanol

Another aspect of the invention is a process for preparing methanol, which comprises contacting the catalyst with a mixture of hydrogen gas and carbon dioxide gas in a reactor.

Usually, the reactor is a tubular reactor, preferably stainless-steel reactor.

Typically, the catalyst is fixed inside the reactor using, for example, glass wool at sides.

The reaction for preparing methanol is carried out usually with a mixture of hydrogen gas and carbon dioxide in a molar ratio $H_2:CO_2 = 3:1$.

Usually, the reaction for preparing methanol may be carried at a temperature comprised between 150 °C and 350 °C, preferably between 200 °C and 300 °C, and more preferably 240 °C.

Typically, the reaction for preparing methanol may be carried at a pressure under 10 bar, preferably under 9 bar, and more preferably under 8 bar.

Generally, in the reaction for preparing methanol the weight hourly space velocity (WHSV) is comprised between 10000 mL.$g_{cat}^{-1}$.$h^{-1}$ and 60000 mL.$g_{cat}^{-1}$.$h^{-1}$, preferably between 12000 mL.$g_{cat}^{-1}$.$h^{-1}$ and 30000 mL.$g_{cat}^{-1}$.$h^{-1}$, more preferably between 15000 mL.$g_{cat}^{-1}$.$h^{-1}$ and 20000 mL.$g_{cat}^{-1}$.$h^{-1}$.

The gas samples may be collected using sampling bags, and the collected methanol may be analysed by gas chromatography, preferably with a flame ionization detector (FID) and helium as the carrier gas, for example. Methanol space time yield (STY), selectivity, and $CO_2$ conversion ($X_{CO2}$) were calculated employing the above mentioned equations.

Use of the catalyst

Another aspect of the invention is use of the catalyst of the invention for the synthesis of methanol by hydrogenation of carbon dioxide.

As disclosed in the Examples section, catalysts of the invention surprisingly show improved yields of methanol, in combination with high stability and selectivity.

The catalyst of the invention demonstrates high methanol STY at all reaction temperatures, and several times higher methanol STY in comparison to the commercially available ternary catalyst $CuZnOAl_2O_3$, as shown in Figure 2.

The stability of the catalyst of the invention is maintained near to 100%, even after a long period of reaction, such as more than 40 hours, which is relatively higher than the stability shown by the commercially available ternary catalyst $CuZnOAl_2O_3$, as shown in Figure 3.

In addition to the stability , the selectivity for the catalyst of the invention is maintained over a long period of time, such as more than 40 hours, whereas for the commercially available ternary catalyst $CuZnOAl_2O_3$, the selectivity decreased gradually after 25 h of reaction, as shown in Figure 4.

The present invention may be defined according to the following embodiments:

1.- A process for preparing the catalyst, which comprises:

1) dissolving a Cu (II) salt and a Zn (II) salt in water,
2) adding biochar (BC) particles to the solution obtained in step 1),
3) stirring the suspension obtained in step 2),
4) adding a precipitant solution to the stirred suspension obtained in step 3),
5) centrifuging and drying the solid obtained in step 4),
6) calcinating the solid obtained in step 5), and
7) reducing with hydrogen gas flow the calcinated solid particles obtained in step 6).

2.- The process according to embodiment 1, wherein Cu(II) salts are selected from Cu(II) nitrate, and hydrates thereof; preferably it is used Cu(II) nitrate hydrate, and more preferably Cu(II) nitrate 3 $H_2O$.

3.- The process according to embodiment 1 or 2, wherein Zn salts are selected from Zn nitrate, and hydrates thereof; preferably it is used Zn nitrate hydrate, and more preferably Zn nitrate 6 $H_2O$.

4.- The process according to any one of embodiments 1 to 3, wherein the precipitant solution is an aqueous solution of an alkali metal carbonate; preferably it is an aqueous solution of sodium carbonate.

5.- The process according to embodiment 4, wherein the concentration of the precipitant is comprised between 0.01 M and 0.1 M, preferably between 0.02 M and 0.05 M, and more preferably it is 0.025 M.

6.- The process according to any one of embodiments 1 to 5, wherein Cu (II) salt and a Zn (II) salt are dissolved in ultrapure water.

7.- The process according to any one of embodiments 1 to 6, wherein the suspension of BC particles and Cu (II) and Zn (II) salts is stirred under vigorous stirring and, preferably, for a time usually about 2 h.

8.- The process according to any one of embodiments 1 to 7, wherein in step 4) precipitant solution is added dropwise.

9.- The process according to embodiment 8, wherein in step 4) the pH of the suspension is adjusted to about 7.0.

10.- The process according to any one of embodiments 1 to 9, wherein the suspension is allowed to age, between step 3) and step 4).

11.- The process according to embodiment 10, wherein the suspension is aged between step 3) and step 4) at a temperature comprised between 50 °C and 70 °C, preferably between 55 °C and 65 °C, and more preferably at 60 °C.

12.- The process according to embodiment 11, wherein the suspension is aged between step 3) and step 4) for a time comprised between 1 h and 5 h, preferably between 1.5 h and 4 h, and more preferably about 2 h.

13.- The process according to any one of embodiments 1 to 12, wherein the suspension prior to the centrifugation of step 5), is washed with water, preferably ultrapure water.

14.- The process according to any one of embodiments 1 to 13, wherein the solid obtained in the centrifugation step is subsequently dried, preferably at a temperature comprised between 100 °C and 110 °C, and more preferably at 105 °C.

15.- The process according to any one of embodiments 1 to 14, wherein calcination takes place in a muffle furnace at a temperature comprised between 300 °C and 500 °C, preferably between 350 °C and 450 °C, and more preferably at 400 °C, and for a period of time comprised between 1 h and 5 h, preferably between 2 h and 4 h, and more preferably 3 h.

16.- The process according to any one of embodiments 1 to 15, wherein the calcinated solid particles are reduced with hydrogen gas flow at a pressure of 1 bar, and 20 sccm (standard cubic centimetres per minute).

17.- The process according to embodiment 16, wherein the reduction step takes place at a temperature comprised between 200 °C and 400 °C, preferably between 250 °C and 350 °C, and more preferably at 300 °C, and for a period of time comprised between 1 h and 4 h, preferably between 1.5 h and 3 h, and more preferably 2 h.

18.- A catalyst obtainable according to the process according to any one of embodiments 1 to 17.

19.- The catalyst according to embodiment 18, wherein the molar ratio Cu:Zn is comprised between 0.5:1 and 5:1, preferably between 1:1 and 4:1, more preferably between 1.5:1 and 3:1, and yet more preferably 2:1.

20.- The catalyst according to embodiment 18 or 19, wherein the content of Cu + ZnO in the catalyst is comprised between 5 wt% and 50 wt%, preferably between 10 wt% and 40 wt%, preferably between 15 wt% and 35 wt%, more preferably between 20 wt% and 30 wt%, more preferably between 22.5 wt% and 27.5 wt%, and yet more preferably the content is 25 wt%, over the total weight of the catalyst.

21.- The catalyst according to any one of embodiments 18 to 20, wherein the content of BC in the catalyst is comprised between 50 wt% and 95 wt%, preferably between 60 wt% and 90 wt%, preferably between 65 wt% and 85 wt%, more preferably between 70 wt% and 80 wt%, more preferably between 72.5 wt% and 77.5 wt%, and yet more preferably the content is 75 wt%, over the total weight of the catalyst.

22.- A process for preparing methanol, which comprises contacting the catalyst according to any one of embodiments 18 to 21 with a mixture of hydrogen gas and carbon dioxide gas in a reactor.

23.- The process according to embodiment 22, wherein the reactor is a tubular reactor; preferably stainless-steel reactor.

24.- The process according to any one of embodiments 22 to 23, wherein the reaction for preparing methanol is carried out with a mixture of hydrogen gas and carbon dioxide in a molar ratio $H_2:CO_2 = 3:1$.

25.- The process according to any one of embodiments 22 to 24, wherein the reaction for preparing methanol is carried at a temperature comprised between 150 °C and 350 °C, preferably between 200 °C and 300 °C, and more preferably 240 °C.

26.- The process according to any one of embodiments 22 to 25, wherein the reaction for preparing methanol is carried at a pressure under 10 bar, preferably under 9 bar, and more preferably under 8 bar.

27.- The process according to any one of embodiments 22 to 26, wherein in the reaction for preparing methanol the weight hourly space velocity (WHSV) is comprised between 10000 $mL.g_{cat}^{-1}.h^{-1}$ and 60000 $mL.g_{cat}^{-1}.h^{-1}$, preferably between 12000 $mL.g_{cat}^{-1}.h^{-1}$ and 30000 $mL.g_{cat}^{-1}.h^{-1}$, more preferably between 15000 $mL.g_{cat}^{-1}.h^{-1}$ and 20000 $mL.g_{cat}^{-1}h^{-1}$.

28.- Use of the catalyst according to any one of embodiments 18 to 21 for the synthesis of methanol by hydrogenation of carbon dioxide.

EXAMPLES

Scanning electron microscopy (SEM) (Merlin FE-SEM) equipped with an energy dispersive spectroscopy (EDS) detector (EDS Oxford LINCA X-Max) was employed to determine the morphology, size distribution and elemental composition of the samples. Nitrogen adsorption/desorption analyses were carried out at -196 °C using an ASAP 2020 Micrometrics Inc (BET analyser). All samples were degassed at 80 °C for 20 h before analysis.

Crystalline structure of the catalyst samples were investigated using Powder X-ray diffraction (XRD). The XRD patterns were recorded in a diffractometer (Panalytical X'Pert) using Cu-K$\alpha$ radiation ($\lambda$=1.5418 Å). The measurements were made at room temperature at a range of 0 °-80° on 2$\theta$ with a step size of 0.02 °. All XRD results were analysed with the simulation of the NPs obtained using the HighScore Plus software.

Preparative example: Preparation of a commercial ternary catalyst

Commercial ternary catalyst $CuZnOAl_2O_3$ (Cu:Zn:Al molar ratio 6:3:1) was prepared using $Cu(NO_3)_2 \cdot 3H_2O$, $Zn(NO_3)_2 \cdot 6H_2O$, and $Al(NO_3)_3 \cdot 9H_2O$ were used as metal sources, according to the process disclosed in Zhang *et al.*, *op. cit.*

Example 1:. Preparation of catalysts

This example discloses the process for preparing the catalyst of the invention, in particular, the preparation of the catalyst having a C:Zn = 2:1 (molar ratio) and having 25 wt% content of Cu plus ZnO, being BC up to 100 wt%, designated as 2-25%CuZnO@BC.

Before the preparation of the catalyst, BC was sieved resulting particles ranging in size from 20 to 100 microns, which were thermally treated at 600 °C for 3 hours to remove the adsorbed water and organic compounds.

1.655 mmol (0.4 g) of $Cu(NO_3)_2.3H_2O$ and 0.827 mmol (0.246 g) of $Zn(NO_3)_2.6H_2O$ were dissolved in 200 mL of ultrapure water. Afterwards, 500 mg of BC were added to the above solution and the mixture was left 2 hours.

Subsequently, the precipitant solution ($Na_2CO_3$, 0.025 M) was added to the dispersion containing the metallic salts and BC, while the solution pH value was adjusted at 7.00.

The solution then was transferred to a 500 mL Scharlau Minireactor HME-R/500 and was allowed to age for 2 hours at 60 °C, to complete the formation of $Cu_xZn_y(OH)_m(CO_3)_n$@BC.

The resultant slurry was washed with ultrapure water many times to eliminate the remaining $Na^+$ from the precipitant solution, it was then centrifuged to obtain a solid, which was dried at 105 °C overnight to obtain the $Cu_xZn_y(OH)_m(CO_3)_n$@BC.

The solid $Cu_xZn_y(OH)_m(CO_3)_n$@BC was calcinated at 400 °C for 3 h, and the calcinated particles were reduced with hydrogen gas flow at 300 °C for 2 hours to obtain the catalyst CuZnO@BC having a molar ratio Cu:Zn = 2:1 and a content of Cu plus Zn of 25 wt% on the BC catalyst.

Following said procedure, the following catalysts were prepared according to the experimental design shown in Table I:

TABLE I

| Example | Cu:Zn molar ratio | (Cu + ZnO) wt% |
|---------|-------------------|----------------|
| 1.1 | 1 | 20 |
| 1.2 | 3 | 30 |
| 1.3 | 1 | 25 |
| 1.4 | 3 | 20 |
| 1.5 | 2 | 20 |
| 1.6 | 1 | 30 |
| 1.7 | 2 | 30 |
| 1.8 | 2 | 25 |
| 1.9 | 3 | 25 |

Further catalysts according to the invention were prepared with the features shown in Table II:

TABLE II

| Example | Cu:Zn molar ratio | (Cu + ZnO) wt% |
|---------|-------------------|----------------|
| 1.10 | 1 | 29 |
| 1.11 | 1 | 37 |
| 1.12 | 1 | 44 |
| 1.13 | 1.5 | 25 |

Catalysts were characterized by scanning electron microscopy (SEM). It was observed that nanoparticles of Cu/ZnO were well dispersed on the surface of BC.

In addition, EDX elemental mapping images of the catalyst samples, obtained using an Energy Dispersive Spectrometer (EDS), confirmed the proper dispersion of Cu/ZnO as well as the excellent mixing of Cu and ZnO nanoparticles, which is critical for a synergistically active catalytic system as far as methanol synthesis by $CO_2$ hydrogenation is concerned.

Example 3: Synthesis of methanol

The synthesis of methanol by hydrogenation of carbon dioxide over the catalyst samples of the invention was conducted in a fixed-bed stainless-steel tubular reactor (5.25 mm internal diameter and 8.90 cm length).

In a typical catalytic experiment, 10 mg of the catalyst was fixed inside the reactor using glass wools at sides.

Prior to the catalytic tests, the catalysts were reduced at 300 °C with hydrogen gas flow (1 bar, 20 sccm) for 2 hours. Then, the temperature was cooled down and the reactant gas, a mixture of $H_2/CO_2$ =3/1 replaced the reductive gas.

The standard reactions were carried out at 240 °C under 10 bar with a weight hourly space velocity (WHSV) of 60000 $mL.g_{cat}^{-1}.h^{-1}$.

The gas samples were collected using sampling bags (SKC FlexFoil PLUS Sample Bag) and the collected methanol was analysed on a gas chromatograph (Shimadzu GC-2010) with a flame ionization detector (FID) and helium as the carrier gas. The software used was Chromeleon to determine the concentrations of chemical compounds, with the inlet temperature of 260 °C and the flow of 50 mL/min, and the detector temperature was 280 °C. An Agilent 7890B GC System chromatograph was employed to measure CO, $CO_2$, and $CH_4$ using a thermal conductivity detector (TCD), and helium as the carrier gas with an inlet temperature of 120 °C, an inlet flow of 20 mL/min, and a detector temperature of 150°C. The software employed was Agilent OpenLAB CDS ChemStation (Version A.01.04).

Methanol space time yield (STY), selectivity, and $CO_2$ conversion ($XCO_2$) were calculated employing the above mentioned equations.

Table III shows the results of the synthesis of methanol, expressed as methanol space time yield (STY, mg $MeOHg_{cat}^{-1}h^{-1}$), for the catalysts prepared in Example 1:

TABLE III

| Example | Cu:Zn molar ratio | (Cu + ZnO) wt% | STY |
|---------|-------------------|----------------|-----|
| 1.1 | 1 | 20 | 226 |
| 1.2 | 3 | 30 | 240 |
| 1.3 | 1 | 25 | 278 |
| 1.4 | 3 | 20 | 246 |
| 1.5 | 2 | 20 | 267 |
| 1.6 | 1 | 30 | 209 |
| 1.7 | 2 | 30 | 260 |
| 1.8 | 2 | 25 | 272 |
| 1.9 | 3 | 25 | 275 |
| 1.10 | 1 | 29 | 217 |
| 1.11 | 1 | 37 | 195 |
| 1.12 | 1 | 44 | 183 |

(continued)

| Example | Cu:Zn molar ratio | (Cu + ZnO) wt% | STY |
|---------|-------------------|----------------|-----|
| 1.13 | 1.5 | 25 | 238 |

All the examples were replicated, obtaining a substantially negligible variability.

Example 4: Comparative experiments

The catalytic performance of the catalyst 2-25%Cu/ZnO@BC was compared with the commercially available ternary CuZnOAl$_2$O$_3$, prepared in the Preparative example, at different reaction temperatures (Figure 2). The catalyst 2-25%Cu/ZnO@BC demonstrated high STY at all reaction temperatures, and at a reaction pressure of 10 bar. Given the reaction conditions of 240 °C and 10 bar as an example, methanol STY of 272 mg MeOHg$_{cat}^{-1}$h$^{-1}$ was obtained for 2-25% Cu/ZnO@BC catalyst which was more than 6 times higher than that of commercially available ternary CuZnOAl2O3¬ (44 mgMeOHg$_{cat}^{-1}$h$^{-1}$).

These values highlighted the significant effect of nanoparticles (NP) dispersion on their catalytic activity.

The stability of 2-25%Cu/ZnO@BC and commercial CuZnOAl$_2$O$_3$ were investigated and compared at 240°C, 10bar, WHSV of 12000 mL gcat$^{-1}$h$^{-1}$, as illustrated in Figure 3. The catalyst 2-25%Cu/ZnO@BC maintained 97% of its catalytic activity after 44 hours of the catalytic reaction, relatively higher than those of the commercial ternary catalyst.

Besides, methanol selectivity values obtained for the catalyst 2-25%Cu/ZnO@BC were relatively higher than those of ternary CuZnOAl$_2$O$_3$: 81.8% for 2-25%Cu/ZnO@ vs 58.4% for CuZnOAl$_2$O$_3$, determined at 240°C, 10 bar, WHSV of 12000 mL gcat$^{-1}$h$^{-1}$ (Figure 4). Methanol selectivity for the catalyst 2-25%Cu/ZnO@BC was maintained after 44 hours, while for the commercial ternary CuZnOAl$_2$O$_3$ decreased gradually after 25 hours of reaction.

**Claims**

1.  A process for preparing the catalyst, which comprises:

    1) dissolving a Cu (II) salt and a Zn (II) salt in water,
    2) adding biochar (BC) particles to the solution obtained in step 1),
    3) stirring the suspension obtained in step 2),
    4) adding a precipitant solution to the stirred suspension obtained in step 3),
    5) centrifuging and drying the solid obtained in step 4),
    6) calcinating the solid obtained in step 5), and
    7) reducing with hydrogen gas flow the calcinated solid particles obtained in step 6).

2.  The process according to claim 1, wherein Cu(II) salts are selected from Cu(II) nitrate, and hydrates thereof; preferably it is used Cu(II) nitrate hydrate, and more preferably Cu(II) nitrate 3 H$_2$O.

3.  The process according to claim 1 or 2, wherein Zn salts are selected from Zn nitrate, and hydrates thereof; preferably it is used Zn nitrate hydrate, and more preferably Zn nitrate 6 H$_2$O.

4.  The process according to any one of claims 1 to 3, wherein the precipitant solution is an aqueous solution of an alkali metal carbonate; preferably it is an aqueous solution of sodium carbonate.

5.  The process according to claim 4, wherein the concentration of the precipitant is comprised between 0.01 M and 0.1 M, preferably between 0.02 M and 0.05 M, and more preferably it is 0.025 M.

6.  A catalyst obtainable according to the process according to any one of claims 1 to 5.

7.  The catalyst according to claim 6, wherein the molar ratio Cu:Zn is comprised between 0.5:1 and 5:1, preferably between 1:1 and 4:1, more preferably between 1.5:1 and 3:1, and yet more preferably 2:1.

8.  The catalyst according to claim 6 or 7, wherein the content of Cu + ZnO in the catalyst is comprised between 5 wt% and 50 wt%, preferably between 10 wt% and 40 wt%, preferably between 15 wt% and 35 wt%, more preferably between 20 wt% and 30 wt%, more preferably between 22.5 wt% and 27.5 wt%, and yet more preferably the content is 25 wt%, over the total weight of the catalyst.

9.  The catalyst according to any one of claims 6 to 8, wherein the content of BC in the catalyst is comprised between 50 wt% and 95 wt%, preferably between 60 wt% and 90 wt%, preferably between 65 wt% and 85 wt%, more preferably between 70 wt% and 80 wt%, more preferably between 72.5 wt% and 77.5 wt%, and yet more preferably the content is 75 wt%, over the total weight of the catalyst.

10. A process for preparing methanol, which comprises contacting the catalyst according to any one of claims 6 to 9 with a mixture of hydrogen gas and carbon dioxide gas in a reactor.

11. The process according to claim 10, wherein the reaction for preparing methanol is carried out with a mixture of hydrogen gas and carbon dioxide in a molar ratio $H_2:CO_2 = 3:1$.

12. The process according to claim 10 or 11, wherein the reaction for preparing methanol is carried at a temperature comprised between 150 °C and 350 °C, preferably between 200 °C and 300 °C, and more preferably 240 °C.

13. The process according to any one of claims 10 to 12, wherein the reaction for preparing methanol is carried at a pressure under 10 bar, preferably under 9 bar, and more preferably under 8 bar.

14. The process according to any one of claims 11 to 13, wherein in the reaction for preparing methanol the weight hourly space velocity (WHSV) is comprised between 10000 $mL.g_{cat}^{-1}.h^{-1}$ and 60000 $mL.g_{cat}^{-1}.h^{-1}$, preferably between 12000 $mL.g_{cat}^{-1}.h^{-1}$ and 30000 $mL.g_{cat}^{-1}.h^{-1}$, more preferably between 15000 $mL.g_{cat}^{-1}.h^{-1}$ and 20000 $mL.g_{cat}^{-1}.h^{-1}$.

15. Use of the catalyst according to any one of claims 6 to 9 for the synthesis of methanol by hydrogenation of carbon dioxide.

Figure 1

Figure 2

Figure 3

Figure 4

Europäisches
Patentamt

European
Patent Office

Office européen
des brevets

# EUROPEAN SEARCH REPORT

**Application Number**

EP 24 18 0709

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 113 877 573 A (INST CHEMICAL IND FOREST PRODUCTS CAF) 4 January 2022 (2022-01-04) * example 2 * | 1-15 | INV.<br>B01J23/80<br>B01J21/18<br>B01J35/40 |
| X | DALEBOUT REMCO ET AL: "Interplay between carbon dioxide enrichment and zinc oxide promotion of copper catalysts in methanol synthesis", JOURNAL OF CATALYSIS, ACADEMIC PRESS, DULUTH, MN, US, vol. 392, 15 October 2020 (2020-10-15), pages 150-158, XP086384850, ISSN: 0021-9517, DOI: 10.1016/J.JCAT.2020.10.006 [retrieved on 2020-10-15] * abstract; page 150 * * page 151, left-hand column, paragraph 2.2 - right-hand column, paragraph 1 * * page 152, left-hand column, paragraph 2.4 * | 1-15 | B01J37/02<br>B01J37/03<br>B01J37/18<br>C07C29/154 |
| X | DEERATTRAKUL VARISARA ET AL: "Influence of reduction time of catalyst on methanol synthesisviaCO2hydrogenation using Cu-Zn/N-rGO investigated byin situXANES", JOURNAL OF THE TAIWAN INSTITUTE OF CHEMICAL ENGINEERS, vol. 80, 18 August 2017 (2017-08-18), pages 495-502, XP085247280, ISSN: 1876-1070, DOI: 10.1016/J.JTICE.2017.08.011 * abstract; page 495 * * page 496, left-hand column, paragraph 2.1 * * page 496, right-hand column, paragraph 2.3 * | 1-15 | **TECHNICAL FIELDS SEARCHED (IPC)**<br><br>B01J<br>C07C |

The present search report has been drawn up for all claims

| Place of search | Date of completion of the search | Examiner |
|---|---|---|
| Munich | 18 November 2024 | Beckmann, Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                                                                                                                                                 
& : member of the same patent family, corresponding document

EPO FORM 1503 03.82 (P4C01)

## ANNEX TO THE EUROPEAN SEARCH REPORT
## ON EUROPEAN PATENT APPLICATION NO.

EP 24 18 0709

This annex lists the patent family members relating to the patent documents cited in the above-mentioned European search report.
The members are as contained in the European Patent Office EDP file on
The European Patent Office is in no way liable for these particulars which are merely given for the purpose of information.

18-11-2024

| Patent document cited in search report | Publication date | Patent family member(s) | Publication date |
|---|---|---|---|
| CN 113877573 A | 04-01-2022 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 1159035 A
- CN 117085689 A
- CN 116037132 A
- CN 114920623 A
- CN 114029063 A
- CN 113877573 A

**Non-patent literature cited in the description**

- **MARKEWITZ et al.** Worldwide innovations in the development of carbon capture technologies and the utilization of CO. *Energy Environ. Sci.*, 2012, vol. 5, 7281-7305
- **LEUNG et al.** An overview of current status of carbon dioxide capture and storage technologies. *Renew. Sustain. Energy Rev.*, 2014, vol. 39, 426-443
- **JADHAV et al.** Catalytic carbon dioxide hydrogenation to methanol: A review of recent studies. *Chem. Engin. Res. Design*, 2014, vol. 92, 2557-2567
- **RAUDASKOSKI et al.** Catalytic activation of CO2: Use of secondary CO2 for the production of synthesis gas and for methanol synthesis over copper-based zirconia-containing catalysts. *Catal. Today*, 2009, vol. 144, 318-323
- **ROHDE et al.** Membrane application in Fischer-Tropsch synthesis to enhance CO2 hydrogenation. *Ind. Eng. Chem. Res.*, 2005, vol. 44, 9653-9658
- **ZHANG et al.** Improved methanol synthesis performance of Cu/ZnO/Al2O3 catalyst by controlling its precursor structure. *Green Energy Environ.*, 2022, vol. 7, 722-781
- **VAN DEN BERG et al.** Structure sensitivity of Cu and CuZn catalysts relevant to industrial methanol synthesis. *Nature Comm.*, 2016, vol. 7, 1-7
- **M. BEHRENS**. Promoting the Synthesis of Methanol: Understanding the Requirements for an Industrial Catalyst for the Conversion of CO. *Angew. Chem. Int. Ed.*, 2016, vol. 55, 14906-14908
- **TOYIR et al.** Highly effective conversion of CO2 to methanol over supported and promoted copper-based catalysts: influence of support and promoter. *Appl. Catal. B Environ.*, 2001, vol. 29, 207-215
- European Biochar Certificate - Guidelines for a Sustainable Production of Biochar.. Carbon Standards International (CSI), 2012